(19) Europäisches Patentamt
European Patent Office
Office européen des brevets

(11) EP 3 177 636 B1

(12) **EUROPEAN PATENT SPECIFICATION**

(45) Date of publication and mention of the grant of the patent:
**13.11.2019 Bulletin 2019/46**

(21) Application number: **15752962.9**

(22) Date of filing: **31.07.2015**

(51) Int Cl.:
***C07K 14/47*** *(2006.01)* ***C07K 1/30*** *(2006.01)*
***C07K 14/495*** *(2006.01)* ***C07K 14/525*** *(2006.01)*
***C07K 14/55*** *(2006.01)* ***C07K 14/57*** *(2006.01)*
***C07K 14/79*** *(2006.01)* ***C07K 1/34*** *(2006.01)*
***C07K 1/36*** *(2006.01)* ***C07K 14/475*** *(2006.01)*
***A61P 17/02*** *(2006.01)* ***A23J 1/20*** *(2006.01)*
***A23C 9/20*** *(2006.01)*

(86) International application number:
**PCT/EP2015/067669**

(87) International publication number:
**WO 2016/020284 (11.02.2016 Gazette 2016/06)**

(54) **PROCESS FOR THE PREPARATION OF HIGH PURITY MIXTURES OF PROTEIN FACTORS FROM BOVINE COLOSTRUM**

VERFAHREN ZUR HERSTELLUNG VON HOCHREINEN MISCHUNGEN VON PROTEINFAKTOREN AUS RINDERKOLOSTRUM

PROCÉDÉ DE PRÉPARATION DE MÉLANGES DE GRANDE PURETÉ DE FACTEURS PROTÉIQUES À PARTIR DE COLOSTRUM BOVIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.08.2014 IT MI20141450**

(43) Date of publication of application:
**14.06.2017 Bulletin 2017/24**

(73) Proprietor: **Tenagro S.r.l.**
**20122 Milano (IT)**

(72) Inventors:
- **CIPOLLETTI, Giovanni**
**I-20143 Milano (IT)**
- **VAGNOLI, Luana**
**I-52100 Arezzo (IT)**
- **CHINI, Jacopo**
**I-50131 Firenze (IT)**
- **BIAGIOLINI, Silvia**
**I-52100 Arezzo (IT)**

(74) Representative: **Valenza, Silvia et al**
**Notarbartolo & Gervasi S.p.A.**
**Corso di Porta Vittoria 9**
**20122 Milano (IT)**

(56) References cited:
**WO-A1-2006/029494 WO-A1-2006/029518**
**WO-A1-2009/135306 WO-A1-2012/087165**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 177 636 B1

## Description

The present invention refers to a process for the preparation of mixtures of protein factors from bovine colostrum and to the mixtures of protein factors from bovine colostrum thus obtained, to the relative compositions comprising said mixtures and said mixtures for therapeutic uses.

It is known that many biological fluids of animal origin such as serum, placenta and colostrum contain essential protein factors such as cytokines, growth factors, chemotactic factors, complement factors, and other components. They are present in different concentrations in the biological fluids of all mammals depending on the species, breed, geographical origin.

The milk and the colostrum of bovine origin (Holstein and Frisian cows) as well as being safe products, are particularly rich of these factors, which are specially lactoferrin, transferrin, IFN$\gamma$, TNF$\alpha$, TGF$\beta$1, IGF-1, immunoglobulins (IgG, IgM and IgA), IL-2 and complement factors C3A and C4A.

The concentration of such factors in the colostrum reaches its maximum 24 hours after delivery to decrease significantly; it is absolutely preferable to collect the colostrum, within the first 6 hours after delivery.

These factors are the same isolable from the supernatant of stem cells culture, thus several therapeutic applications of the extracts or the isolated fractions from mammal colostrum have been proposed, as alternative treatment to the costly and difficult stem cell therapy (for a review of this clinical uses, see references [1] and [2]).

However, such extractive process from biological fluids of bovine origin show different critical elements because they are applied to an heterogeneous product such as milk or colostrum.

Among the main drawbacks there is the need to preliminarily eliminate caseins that may be responsible for allergic phenomena or hinder the isolation of other desirable proteins which are smaller and less abundant. Caseins are traditionally removed upon coagulation (precipitation) and subsequent filtration.

Another critical element is the presence of reducing sugars, such as, for example, glucose and lactose, which may create covalent adducts with the protein constituents, with consequent loss of the protein functionality and induction of harmful responses. The subject reaction foresees, as first step, a Schiff base formation by condensation of the carbonyl moiety of the reducing sugar with the primary amine moiety of the lysine or with the end amine group of the protein. Once formed the Schiff base spontaneously rearranges, with the formation of stable adducts (Maillard reaction). Furthermore, the sugars may undergo an oxidative process with the formation of carbonyl reactive species such as, for example, glyoxal, methylglyoxal and deoxyglucosone, able to react with the nucleophile sites of the proteins (histidine, lysine, arginine, cysteine) thus forming an heterogenous mixture of reaction products among which carboxylysin-, carboxycystein- and imidazoline derivatives. The proteins modified by the reducing sugars or by their degradation products derive their name from the advanced glycation products or AGEs (Advanced glycation end products, ref. [3]).

The formation of modified proteins, further being induced by reducing sugars, is also promoted by the products of lipidic oxidation.

More in detail, the oxidative degradation of the lipids and in particular of the polyunsaturated fatty acids (PUFA), involves the formation of carbonyl reactive species (RCS) such as, for example, the alpha, beta-unsaturated aldehydes (hexenal, hydroxynonenal, acrolein), the dialdehydes (malondialdehyde) and ketoaldehydes, form Schiff s bases and Michael's adducts with the protein substrates. The protein adducts formed by covalent addiction of proteins with aldehyde compounds generated by lipid peroxidation are named ALEs (Advance Lipoxidation End Products, ref. [4])

The formation of AGEs and ALEs within a complex proteoma like the colostrum, may cause a sequence of biological responses potentially detrimental and in the meantime alter the biological functions of the same proteins. More particularly, when the originally bioactive protein is covalently modified with the formation of AGEs and ALEs, its biological function is irreversible compromised and this may be due to different mechanisms, as the altered conformation, the covalent alteration of the active site and aggregate-formation. AGEe and ALEs further inducing functionality loss, are potentially harmful as they may be immunogenic and act as RAGE receptor agonists by inducing inflammatory and fibrotic processes, as well as induce the outcome of the oxidative process by activation of NADH oxidase (see references [5],[6]).

AGEs and ALEs formation in the colostrum, milk and derivatives thereof (for example powder and long term storage milk) is driven by air exposure, temperature and storage. The presence of AGEs and ALEs in such matrices is directly evaluated by different analytical methods such as for example immunochemistry analysis (Western blot and ELISA assays) and mass spectrometry ([7],[8]).

A well-known marker of AGEs formation which may be routinely assessed is represented by furosine (or $\varepsilon$-furoylmethyl-lysin), which is a product of acid hydrolysis of AGEs:

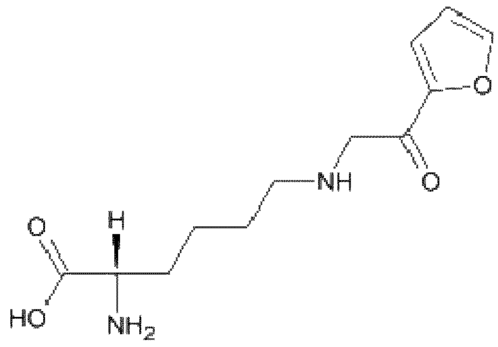

Furosine

Both the international patent applications WO2013/098331 and WO2013/098333 (references [9],[10]) disclose a process for the production of mixtures of biological factors from bovine colostrum. However, the disclosed process, does not foresee any reduction step of the lactose content initially present in the colostrum thus preventing the formation of AGEs in the final product.
In addition, the casein content is reduced by a passage through a ceramic ultrafiltration membrane of 300,000 Da. The outermost criticality of such an operation has been observed from a pilote scale experiment, that in presence of an high heterogeneous product (with a fatty content derived from a centrifuge step operated at a temperature of 20-25°C) leads to a tangible reduction of permeation of the active proteins, with consequent reduction of the final yield.
WO2011/064114 (ref. [11]) discloses a process to obtain a mixture of biological factors deriving from bovine colostrum (Holstein and Frisian breed) through an initial step of microfiltration with a molecular cut-off of 2-6 micron which allows the separation of fats and large sized proteins such as caseins and lactalbumins, from the soluble serum proteins like the biological factors of interest. As outlined in the same document, the efficacy of such extraction may be remarkably reduced due to the formation of lipid and colloidal caseins layers on the surface of the microfiltration membrane with consequent obstruction of the pores of the membrane itself and loss of active factors which would remain retained in the retentate (see page 19, WO2011/064114). In addition, the microfiltration step allows the removal of the lactalbumin fraction as well as the lipid and casein fractions. As the lactalbumin has a molecular weight of 14,2 KDa, this means that also other active proteins, for example, IGF (7.5 kDa), EGF (9 kDa), IL-17 (14 kDa) showing a molecular weight of the same size order or lower than lactalbumin will be eliminated. Afterwards, the permeate obtained in the microfiltration step is concentrated by tangential nanofiltration through a membrane having a molecular cut-off of 400 Da. The employed molecular cut-off would not be anyway suitable for the passage of lactose in the permeate. Therefore, the whole extractive process according to WO2011/064114 does not disclose any step of reduction of the lactose content and consequently of the AGEs formation. WO2012087165 discloses enriched dairy powder produced from bovine colostrum several steps, including crystallisation for the removal of lactose.
WO2009135306 discloses another method for the preparation of a mixture from bovine colostrum, involving heating and ultrafiltration steps.
Consequently, there is a great interest towards the development of processes for the extraction and purification of bovine colostrum fractions that contain protein factors unchanged from the biological activity point of view, and are devoid of undesirable substances, like AGEs and ALEs.
The authors of the present invention have now developed a process for the preparation of mixtures of protein factors from bovine colostrum having an advantageous quality and safety profile, being in particular characterized by:

1) enhancement of the content of active protein factors in the final product;
2) reduced content of lactose in the final product (<1%);
3) reduced content of AGEs (furosine) and ALEs in the final product.

Additionally, the process of the invention may foresee a single elimination step of the casein and lipid components with consequent increase of the final yield and overall reduction of the manufacturing time.
The mixtures of protein factors from bovine colostrum thus obtained show a reduced immunogenicity, in addition to a greater concentration of active protein factors whose activity is not compromised by the glycation and carbonylation events.
It is therefore an object of the present invention a process for the preparation of mixtures of protein factors from bovine colostrum comprising the following steps:

a) dilution of bovine colostrum, skimming and precipitation of casein at pH comprised between 4 and 6; preferably at isoelectric pH of casein (4.6);
b) centrifugation to remove the lipid and casein components, preferably at 4000 rpm;
c) concentration and diafiltration on polymer membrane with a molecular cut-off between 1,000 to 20,000 Da, preferably 4,000 Da, to reduce the lactose content;
d) adjusting the pH around neutrality (that is in the range 6.5 ± 1) and centrifugation, preferably at 8500 rpm;
e) filtration;
f) drum filling and freezing;

wherein the step c) is repeated to obtain a lactose content limit ≤1% calculated with respect to dry weight, i.e. as HPLC lactose concentration/dry weight x 100.

According to an alternative embodiment of the process according to the invention it is possible to foresee a step g) of freeze-drying the final product, once frozen.

Preferably, the filtration step e) comprises a prefiltration step carried out on filters of porosity comprised between 0.5 μm and 2 mM, preferably of 0.5 μm, to facilitate the subsequent sterilizing filtration carried out with filters of porosity comprised between 0.45 and 1 μm, preferably of 0.2 μm.

According to a preferred embodiment of the present invention the critical steps a) and c) of the process are carried out in the following conditions:

a) Dilution of the colostrum, skimming and precipitation of casein:

The colostrum is diluted with a volume of water (or saline) comprised between 0.5 and 100 volumes, preferably 1-10 volumes, more preferably 1 volume. The suspension is heated between 10-60°C, preferably at 38°C. Then, caseins are precipitated shifting the pH of the suspension between 4-6, preferably to 4.6 (isoelectric pH of casein). Casein precipitation is followed by a centrifugation step.

The casein component is eliminated by precipitation at pH corresponding to the casein isoelectric point and then centrifuged. In this way it is possible to obtain a supernatant with an high content of active factors and consequent enhancement of the final yield.

The essential factors present in the starting colostrum remained almost unchanged in the supernatant obtained after centrifugation which is conveyed to the next step.

c) Concentration and diafiltration on ultrafiltration polymeric membrane between 1000 and 20000 Da, preferably 4000 Da:

In such step, the previously skimmed colostrum devoid of the casein component, is firstly concentrated and afterwards undergoes dialysis against water.

This operation (diafiltration or dialysis), together with the use of a membrane having a cut-off suitable for the lactose passage in the permeate, allows the reduction up to the desired threshold. Such operation is driven up to the achievement in the retentate of a lactose value less or equal to 1% calculated on the dry weight, that is as HPLC lactose concentration/dry weight x 100.

The substantial reduction of the lactose content, allows to drastically reduce the formation of AGEs in the final product as well as the skimming avoids ALEs formation.

Moreover, also Prolin-rich polypeptides (PRP) and sialic acid will not be present in the final product (detection limit of 1%) as they will be isolated by the diafiltration employing membranes with a cut-off of 4 kDa (see Example 5).

It is a further object of the present invention a mixture of protein factors from bovine colostrum having a lactose content ≤ 1% with respect to dry weight. Such mixture of protein factors it is further characterized by a protein content higher than 90% (calculated by Kjeldahl method).

In addition the invention contemplates a pharmaceutical composition comprising such mixture of protein factors from bovine colostrum together with further pharmaceutically acceptable excipients and/or adjuvants.

Preferably, the above pharmaceutical composition is suitable for topical application or for oral administration.

It is another object of the present invention a pharmaceutical composition for topical application comprising such mixture of protein factors from bovine colostrum together with further pharmaceutically acceptable excipients and/or adjuvants, for the treatment of pathological conditions requiring the tissue repair or regeneration.

According to a preferred embodiment of the present invention the above pathological conditions comprise ulcers or cutaneous lesions, burns, oral lesions, corneal lesions, nasal mucosal lesions, Herpes virus mediated lesions and wound healing.

The pharmaceutical compositions for topical application according to the invention are preferably in the form of a cream, ointment, powder, gel, lotion, mouth washing, tampon, transdermal patches.

Finally, the invention refers to a dietary supplement comprising the mixture of protein factors from bovine

colostrum, optionally in association with one or more further ingredients which are acceptable from the dietary point of view.

The present invention will be now disclosed for illustrative but not limitative purpose, according to a preferred embodiment with particular reference to the enclosed drawings wherein:

- Figure 1 shows a flow scheme of the process for the preparation of the mixtures of protein factors from bovine colostrum according to the present invention;
- Figure 2 shows the Western blot analysis carried out on three different lots (Q194/(1)47, Q194/(1)48, Q194/(1)49) using polyclonal antibodies against carboxymethyl-lysine and the adducts of hydroxynonenal (ALEs marker); as a positive control carboxymethyl-lysine (CML-BSA) and HNE (HNE-BSA) modified albumins have been used;
- Figure 3 shows the brightfield microscope imagines of the morphological evaluation of untreated cellular monolayer (panel A), Gel formulation 1 treated cell monolayer (panel B), Gel formulation 2 treated cell monolayer (panel C);
- Figure 4 illustrates the murine model of pressure ulcers by using externally placed magnets to create the ischemic events of ischemia reperfusion (IR) injury;
- Figure 5 shows the imagines of the skin macroscopic analysis of dorsal skin of the mice control group after 3 IR cycles revealing the presence of mild skin lesions, oedema and signs of necrosis of the epidermis;
- Figure 6 shows the imagines of the histopathological analysis of dorsal skin samples from the mice of the control group stained by hematoxylin-eosin;
- Figure 7 shows the imagines of the skin macroscopic analysis of dorsal skin of the mice group treated with the cream containing the bovine colostrum extract 4% of the invention;
- Figure 8 shows the imagines of the histopathological analysis of dorsal skin samples from the mice group treated with the cream containing the bovine colostrum extract 4% of the invention;
- Figure 9 shows the LC-ESI-MS analysis of the PRPs profile in the permeate of the lactose-free mixture of protein factors from bovine colostrum deriving from the step of ultrafiltration and diafiltration through a 4000 Da cut-off membrane.

EXAMPLE 1: *Process for the extraction of mixtures of protein factors from bovine colostrum with a reduced content of AGEs and ALEs*

Starting material

Bovine colostrum from Holstein cows has been collected within the first 6 hours after calving. Subsequently, it is freezed and stored at -20°C.

Productive process steps

Figure 1 shows an illustrative scheme of the process steps according to the present invention, that may be summarized as follows:

- Dilution with water of the defrozen colostrum (equal volume)
- Skimming and precipitation of casein at isoelectric point (pH 4.60)
- Centrifugation at 4000 rpm with Alfa Laval mod. FL118 centrifuge to eliminate the precipitated fat and casein
- Reduction of the lactose content: concentration and diafiltration on 4000 Da polymeric membrane
- pH adjustment at 7.0 $\pm$ 0.2
- Centrifugation at 8500 rpm with Alfa Laval mod. LAB 102 B-25 centrifuge
- Prefiltration on 0.5 $\mu$m cartridge filter and filtration on 0,2 $\mu$m absolute filter to remove possible microorganisms (i.e. bacteria, moulds and yeasts)
- Packaging
- Freezing at -20°C
- Freeze-drying

Dilution of colostrum with water, skimming and precipitation of casein

A 250 liter inox steel reactor equipped with stirring system, thermostat and probe for measuring pH, is filled with 100 Kg of bovine colostrum (stored at -20°C and preventively defrozen at room temperature, 20-25°C) and diluted with one volume of drinking water thus obtaining a 200 liters suspension. Such suspension is heated, under stirring, at a temperature of 38 $\pm$ 1°C. After about 1 h, casein precipitation is carried out by acidification up to pH 4.6. After 1 h stirring, centrifugation is carried out.

Centrifugation at 4000 rpm to eliminate fat and casein precipitates

The reactor is discharged and centrifugation is carried out to eliminate all the precipitates (fat and caseins).

Reduction of the lactose content and diafiltration on a 4000 Da polymeric membrane

The clear fraction separated by centrifuge is concentrated using ultrafiltration membranes with a molecular cut-off of 4000 Da in order to allow the passage of lactose into the permeate and to retain the protein component into the concentrate.

The retentate is concentrated up to a volume of about 1:3 with respect to the initial volume; after diafiltration against water is carried out until the desired lactose content in the retentate is achieved (less or equal to 1% with respect to dry weight).

The lactose content in the solution is determined by HPLC (Perkin Elmer with Transgenomic ICE SEP Ion 300 column, 0.015 N sulphuric acid eluant, temperature 45°C, flow rate 0.4 ml/min).

pH adjustment and centrifugation at 8500 rpm

pH of the diafiltered retentate is adjusted to neutrality, followed by centrifugation at 8500 rpm with Alfa Laval Mod. mod. FL118 centrifuge to clarify the solution before the filtration step.

0.5 μm prefiltration and 0.2 μm filtration

The solution is filtered with 0.5 μm absolute cartridge filters (i.e. Sartorius, Millipore, Pall, etc.) and if necessary with further 0.2 μm absolute cartridge filters (i.e. Sartorius, Millipore, Pall, etc.).

Drum filling and freezing

The filtered product is directly conveyed to the collecting container for the storage or optionally for the freeze-drying.

Freeze-drying

The frozen product undergoes a process of freeze-drying starting from a temperature of - 50°C and stopping at a temperature of 25-30°C.

EXAMPLE 2: *Quali-quantitative analysis of the final product by the process of the invention*

MATERIALS AND METHODS

Immunoglobulins, growth factors and complement factors dosages have been measured by ELISA assay.

For the measurement of the lactose and furosine levels, an HPLC analysis has been carried out.

RESULTS

The results of the analysis carried out on the freeze-dried product deriving from three different lots (Q194/47, Q194/48, Q194/49) obtained through the process disclosed in Example 1, are illustrated in the following Table 1.

TABLE 1

| Lot | Q194/(1)47 | Q194/(1)48 | Q194/(1)49 |
|---|---|---|---|
| Lactoferrin μg/mg liophilic | 6.7 | 5.7 | 9.3 |
| Transferrin μg/mg liophilic | 1.1 | 1.8 | 1.3 |
| IgA mg/mg liophilic | 0.19 | 0.1 | 0.13 |
| IgM mg/mg liophilic | 0.2 | 0.3 | 0.23 |
| IgG mg/mg liophilic | 0.6 | 0.67 | 0.6 |

(continued)

| Lot | Q194/(1)47 | Q194/(1)48 | Q194/(1)49 |
|---|---|---|---|
| IGF ng/mg liophilic | 2.0 | 0.8 | 2.2 |
| TGFβ pg/mg liophilic | 9.8 | 12.8 | 20.9 |
| IFN-γ pg/mg liophilic | 3.2 | 9.0 | 2.3 |
| TNFα pg/mg liophilic | 26 | 66 | 25 |
| IL-2 pg/mg liophilic | 2.3 | 5.2 | 2.3 |
| C3A pg/mg liophilic | 1.2 | 2.1 | 1.1 |
| C4A pg/mg liophilic | 1.1 | 0.7 | 1.5 |

Such mixture of protein factors is further characterized by a protein content greater than 90% (calculated by Kjeldahl method).

Results relative to the lactose and furosine dosages assayed on the three lots are depicted in the following Table 2.

TABLE 2

| | Q194/(1)47 | Q194/(1)48 | Q194/(1)49 |
|---|---|---|---|
| Lactose | 0.06% | 0.26% | 0.56% |
| Furosine | 42.3 ± 5.7 mg\100 g protein | 56.3 ± 6.4 mg/100 g protein | 55.0 ± 6.3 mg/100 g protein |

From the analysis of the above Table 2 it can be inferred a lactose content less than 1% and an extremely reduced furosine content.

To further confirm the effectiveness of the proposed process in order to reduce the formation of AGEs and ALEs, a Western blot analysis on the above three lots has been carried out.

The analysis foresees the SDS-PAGE electrophoretic separation of the proteome, followed by gel blotting on nitrocellulose membrane and subsequent identification of AGEs and ALEs using anti-carboxymethyl-lysine polyclonal antibodies (AGEs marker) and the hydroxynonenal adducts (ALEs marker). As positive controls carboxymethyl-lysine (CML-BSA) and HNE (HNE-BSA) modified albumins have been used.

The results depicted in Figure 2 show the absence of any band referable to CML and HNE protein adducts in the three analysed lots. On the contrary, the standard bands are well visible.

EXAMPLE 3: *In vitro comparative evaluation of wound healing activity of gel formulation comprising the mixture of protein factors of the invention*

The purpose of this test is to compare the efficacy of gel formulations comprising the mixture of protein factors of the invention in the repairing of wounds simulating this situation *in vitro* by making a cut on cell monolayers of human fibroblasts and then comparing the approximation of the edges of the cut in treated and untreated cells.

MATERIALS AND METHODS

Cell lines

Human fibroblasts from derma cell line (HuDe) was employed for MTT assay and purchased by Istituto Zooprofilattico sperimentale della Lombardia e dell'Emilia Romagna, Brescia, Italy.

Negative control (C-): untreated HuDe cells.
Quality control (QC): internal control consisting of 15% New Born Calf Serum (NBCS) with known wound healing activity. Cells were grown till they reach confluence, the monolayer has been lesioned and finally the cells have been cultivated in a medium containing foetal calf serum enriched with fibroblasts growth factors (15%).

Tested gel formulations:

The following gel formulations with or without the bovine colostrum extract according to the invention have been prepared and tested.

- Gel Formulation 1 with colostrum extract (Internal Code AI13-003D):

| **INCI Name** | **% p/p** |
|---|---|
| Water | 89,250 |
| Aloe barbadensis leaf juice | 2,000 |
| **Bovine colostrum extract** | 1,800 |
| Hydroxiethylcellulose | 1,500 |
| Xantan gum Chondrus Crispus (Carrageenans) | 1,100 |
| Polycarbophil AA1 | 0,800 |
| Phenoxiethanol | 0,750 |
| Sodium Hydroxide | 0,500 |
| Melissa officinalis glycolic extract | 0,500 |
| Sodium benzoate | 0,500 |
| Potassium sorbate | 0,500 |
| Sucrose | 0,300 |
| Sodium saccharine | 0,200 |
| Imidazolidinyl Urea | 0,200 |
| Aroma | 0,100 |

- Gel formulation 2 (without actives):

| **INCI Name** | **% p/p** |
|---|---|
| Water | 91,05 |
| Aloe barbadensis leaf juice | 2,000 |
| Hydroxiethylcellulose | 1,500 |
| Xantan gum Chondrus Crispus (Carrageenans) | 1,100 |
| Polycarbophil AA1 | 0,800 |
| Phenoxiethanol | 0,750 |
| Sodium Hydroxide | 0,500 |
| Melissa officinalis glycolic extract | 0,500 |
| Sodium benzoate | 0,500 |
| Potassium sorbate | 0,500 |
| Sucrose | 0,300 |
| Sodium saccharine | 0,200 |
| Imidazolidinyl Urea | 0,200 |
| Aroma | 0,100 |

Preliminary MTT assay

In order to select the concentrations of the gel formulations to be used for the comparative test (not cytotoxic concentrations for the cells) a preliminary MTT assay of cell cultures of fibroblasts (Hude) was also performed.

Cells were treated with scaled concentrations of the two gel formulations with or without actives (as low as 1 mg/ml and subsequent dilutions 1:2) and untreated cells were used as negative control.

The MTT assay is performed on cells treated with gel formulations having the following different concentrations: 1 mg/ml - 0.25 mg/ml and 0.15 mg/ml.

A suitable number of cells is spread in the wells (96 wells plate, 150 μl/well of cell suspension).

Once a confluence of 60-70% has been reached, fresh medium is added with scalar dilutions of tested gel formulations (starting from 1 mg/ml and subsequent dilution 1:2).

Incubation with the different gel formulations goes overnight (24 hrs). After medium substitution with fresh

medium + MTT, cells are incubated for 3 h at 37°C. Then cells are washed more times to eliminate MTT solution wastes.

The fluorimeter reading is made at 540 nm wavelength.

Wound simulation

After making a cut on the cell monolayer of confluent fibroblasts (simulation of a wound) through the use of a needle of insulin syringe to simulate a wound, the cells were treated with the two gel formulations having the chosen concentration, as negative control untreated cells were used and, as internal quality control one standard with known activity of wound healing activity.

Therefore, the following evaluations have been performed:

- a morphological evaluation of the monolayer
- MTT assay
- interleukin IL8 dosage.

Morphological evaluation

The morphological evaluation of the monolayer has been carried out by acquisition of bright field microscope photos to check the thinning/closing of the cut with approach/confluence of the flaps.

MTT assay

The test is blocked (after about 96 hours), and an MTT assay is performed to assess cells viability. The MTT assay is performed on cells treated with concentrations of product choices equal to 1-0.25 mg/ml and 0.15 mg/ml.

IL8 assay

The dosage of the IL8 is made on supernatant from both untreated cells and treated cells with gel formulations having the following different concentrations 1 mg/ml-0.25 mg/ml and 0.15 mg/ml.

RESULTS

Preliminary cell viability assay

Results are given as Optical Density (O.D.) that is proportional to alive cells. It was then calculated the % of cell viability according to the formula:

$$\frac{\text{(average O.D. sample)}}{\text{(average O.D. control)}} * 100$$

Based on the obtained results, concentrations of 1 mg/ml-0.25 mg/ml and 0.15 mg/ml in the gel formulations with or without active ingredients were chosen to carry out the MTT assay. Tables 3 and 4 depicted the O.D. results obtained with Gel formulation 1 and 2 in the whole range of concentrations analysed.

TABLE 3: Gel formulation 1 with colostrum extract

| Sample (mg/ml) | 1 | 0,5 | 0,25 | 0,125 | 0,062 | 0,031 | 0,015 | 0,0078 | Negative control |
|---|---|---|---|---|---|---|---|---|---|
| O.D. | 0,54 | 0,52 | 0,537 | 0,55 | 0,55 | 0,55 | 0,49 | 0,58 | 0,55 |
| % of cell viability | 97,5 | 94,6 | 97,1 | 99,30 | 99,4 | 99,8 | 89,8 | 105,40 | 100,00 |

TABLE 4: Base gel formulation 2 without extract

| Sample mg/ml | 1 | 0,5 | 0,25 | 0,125 | 0,0625 | 0,0312 | 0,0156 | 0,0078 | Negative control |
|---|---|---|---|---|---|---|---|---|---|
| O.D. | 0,54 | 0,56 | 0,56 | 0,57 | 0,57 | 0,58 | 0,60 | 0,61 | 0,593 |

(continued)

| Sample mg/ml | 1 | 0,5 | 0,25 | 0,125 | 0,0625 | 0,0312 | 0,0156 | 0,0078 | Negative control |
|---|---|---|---|---|---|---|---|---|---|
| % of cell viability | 91,4 | 95,0 | 94,9 | 95,8 | 95,9 | 97,2 | 101,5 | 102,9 | 100 |

From the morphological evaluation a net approach of the flaps of the monolayer was observed in the plates treated with the various concentrations of Gel formulation 1 compared to the plates with the untreated cells and the plates treated with Gel formulation 2 (see Figure 3, panels A-C). Figure 3A shows that no approximation of the edges of the cut is observed in the untreated cells monolayer; figure 3B shows that an evident approximation of the edges of the cut with almost total closure of the same is observed in the cells treated with Gel Formulation 1 (with actives); Figure 3C shows that any approximation of the edges of the cut is observed in the cells treated with Gel Formulation 2 (no actives).

The MTT assay showed an increase in cell viability of fibroblasts in the plates treated with Gel Formulation 2 to indicate the effectiveness of the base formulation without actives in the nourishment of the cell, while the viability of fibroblasts in the plates treated with Gel Formulation 1 has proved to be comparable to that of untreated cells, indicating the total absence of cytotoxicity of the tested medical device and to demonstrate a modulation of the metabolism of the cells that resulting healthy and viable and capable to reconstruct the monolayer where the cut has been made (see Figure 3).

Results of the MTT assay are given as Optical Density (O.D. that is proportional to alive cells) and depicted in Tables 5 and 6. QC is the quality control, that is an internal control with known wound healing activity:

It was then calculated the % inhibition of cell viability according to the formula:

$$\%\ \text{inhibition} = 100 - \frac{(\text{average O.D. sample})}{(\text{average O.D. control})} * 100$$

TABLE 5: Gel formulation 1

| Sample mg/ml | 1 | 0,25 | 0,15 | Negative control | QC |
|---|---|---|---|---|---|
| Average O.D. | 1,419 | 1,473 | 1,648 | 1,586 | 2,566 |
| % of cell vitality | 89,50 | 92,87 | 103,89 | 100,00 | 161,78 |

TABLE 6: Gel formulation 2

| Sample mg/ml | 1 | 0,25 | 0,15 | Negative control | QC |
|---|---|---|---|---|---|
| Average O.D. | 2,048 | 1,628 | 1,590 | 1,586 | 2,566 |
| % of cell vitality | 129,12 | 102,63 | 100,28 | 100,00 | 161,78 |

Evaluation of anti-inflammatory activity

Results of IL-8 cytokine assay are given as Optical Density (O.D.) and pg/ml, that are proportional to induced IL-8.

The % of reduction of interleukin 8 in the samples is calculated by the following formula:

$$\% = 100 - \frac{(\text{pg/ml IL8 sample})}{(\text{pg/ml IL8 control})} * 100$$

IL8 dosage showed significant decrease in the % of IL8 to concentrations of 1 and 0.25 mg/ml by both tested gel formulations, showing therefore a comparable anti-inflammatory action on fibroblasts.

Results are depicted in the following Tables 7 and 8.

TABLE 7: Gel formulation 1

| Sample | Negative control | 1 mg/ml | 0,25 mg/ml | 0,15 mg/ml |
|---|---|---|---|---|
| O.D. Mean value | 1.9 | 1.292 | 1.659 | 1.755 |
| pg/ml Mean value | 3.064,727 | 1.959,970 | 2.627,693 | 2.838,670 |
| % IL8 | 100 | 63.95 | 85.74 | 92.62 |
| % reduction of IL8 | 0 | 36.05 | 14.26 | 7.38 |

TABLE 8: Gel Formulation 2

| Sample | Negative control | 1 mg/ml | 0,25 mg/ml | 0,15 mg/ml |
|---|---|---|---|---|
| O.D. Mean value | 1.9 | 1.392 | 1.623 | 1.779 |
| pg/ml Mean value | 3.064,727 | 2.140,429 | 2.560,639 | 2.844,373 |
| % IL8 | 100 | 69.84 | 83.55 | 92.81 |
| % reduction of IL8 | 0 | 30.16 | 16.45 | 7.19 |

These results overall indicate that the effectiveness of the active ingredients of the colostrum extract present in the Gel formulation 1 have a different target than the reduction of the inflammatory response.

The obtained results have showed the effectiveness in wound healing of the Gel formulation 1, compared to the same basic formulation without actives (Gel Formulation 2).

In fact, Gel formulation 2 showed a "nutrient" activity for fibroblasts in vitro but it was not able to stimulate wound healing (simulation in vitro by cutting the monolayer of cultivated fibroblasts and no evidence of the approximation of the edges of the cut), activity that is due to the presence of the active protein factors present in the Gel formulation 1.

EXAMPLE 4: *Evaluation of the protective effect of the topical use of the extract of bovine colostrum of the invention in a murine model of pressure ulcers*

MATERIALS AND METHODS

Tested 4% cream formulation:

The following cream formulation containing the bovine colostrum extract (4%) according to the invention has been prepared and tested.

| **INCI Name** | **% p/p** |
|---|---|
| Water | 67,420 |
| C12-15 Alkil Benzoate | 5,000 |
| Glyceryl Stearate (and) PEG-100 Stearate | 5,000 |
| PP013 AIM Colostrum | 4,000 |
| Octyl stearate | 4,000 |
| Cetearyl Alcohol | 3,000 |
| Prunus amygadalus dulcis oil | 3,000 |
| Glycerin | 3,000 |
| Cyclomethicone | 2,000 |
| Cera Alba | 1,000 |
| Tocopheryl Acetate | 1,000 |
| Phenoxiethanol | 0,750 |

(continued)

| INCI Name | % p/p |
|---|---|
| Aluminuim starch octenylsuccinate | 0,350 |
| Imidazolidinyl urea | 0,280 |
| Disodium EDTA | 0,200 |

Animals

10 weeks-old CD-1 female mice were employed (in the number of 8 animals) supplied by Charles River, Calco, Italy. The weight was between 20-25 g at the starting of the test.

Caging

Each mouse was caged in cages of mm 160x270x370 for two weeks before the starting of the test.
Automatic control assured 12 hours light, 12 hours dark.
Room temperature and humidity were regulated by a conditioning plant.

Cleaning and disinfection

The cages and the housing room were cleaned before the animals were accommodated, then cleaning and disinfection were performed twice a week.

Feeding

Animals were fed ad libitum with standard pelletized diet, provided by Laboratorio Dottori Piccioni (Italy).

Watering

Animals were watered with purified water, available ad libitum and distributed via an automatic watering system.
Animals selected for the study were singularly housed in 14 numbered cages.
Animals used in the study were randomly assigned to the different treatment groups at the beginning of the study.

Study design

The study reports the development of a murine model of pressure ulcers by using externally placed magnets to create the ischemic events of ischemia reperfusion (IR) injury.
The animals were individually housed, their backs have been shaved, cleaned with alcohol, the skin has been gently pulled up and placed between two round ceramic magnetic plates which have a 12-mm diameter (113 $mm^2$) and are 5 mm thick, with an average weight of 2.4 g and 1,000 G magnetic forces; this process creates a compressive pressure of 50 mmHg between the two magnets (see Figure 4).
Then the animals have been divided into two groups as follows:

**1) Control group:** three IR cycles have been performed in 3 mice to initiate decubitus ulcer formation. A single IR cycle consists of a 12-hour period of magnet placement, followed by a release of rest period of 12 hours. After the 3 IR cycles, the animals have been sacrificed. Group B: three IR cycles have been performed in each mouse to initiate decubitus ulcer formation. A topical administration of 200 mg of the cream comprising 4% colostrum bovine extract of the invention has been applied on the backs of each mouse the day before the first IR cycle and at the end of each compressive cycle.

After the 3 IR cycles, the animals have been sacrificed. Skin samples of each mouse have been collected from the treated area, fixed in 10% phosphate-buffered formalin and wax embedded. 2 μm thickness sections were obtained and collected on silanizated slides and stained by hematoxylin-eosin. The samples were then observed with an optical microscope Nikon 80i, fitted with a digital camera.

RESULTS

Skin macroscopic analysis

Skin macroscopic analysis of dorsal skin of the control group (after 3 IR cycles) revealed the presence of mild skin lesions, oedema and signs of necrosis of the epidermis (see Figure 5).

Histopathological analysis

Histological analysis of dorsal skin samples stained by hematoxylin-eosin showed: wide ulcerative area of the epidermis; marked spongiosis of basal layer (intercellular bridges appear very prominent); marked diffuse inflammatory infiltrate; connettival oedema; vascular hyperemia; presence of extravasal eritrocytes (see Figure 6).

**2) Group B:** 3 IR cycles + Cream formulation 4% at the end of each compressive cycle and the day before the first IR cycle.

Skin macroscopic analysis

Macroscopic analysis of dorsal skin revealed: skin ulcers with necrosis areas and oedema (see Figure 7).

Histopathological analysis

Histological analysis of dorsal skin samples stained by hematoxylin-eosin showed: wide ulcerative area of the epidermis; when present epidermis is hyperplasic, with not regular thickness, marked spongiosis of basal layer and presence of lymphocytes; marked diffuse inflammatory infiltrate; connettival oedema; vascular hyperemia; presence of extravasal eritrocytes (see Figure 8).

EXAMPLE 5: *Evaluation of the presence of the Prolin-rich polypeptides (PRPs) in the preparation containing the mixture of protein factors from bovine colostrum produced according to the process of the invention*

Prolin-rich polypeptides (PRPs) contained in the bovine colostrum are polypeptides enriched in proline (about 22%) and acidic amino acids, while they are poor of alanine, glycine, arginine, methionine and histidine and lack tryptophan and cysteine [12].

Initially PRP, further defined also Clostrinin (CLN), were identified as a single protein with a molecular weight of about 17-18 kDa. Further studies allow a better PRP characterization that result to be made of at least 32 polypeptides with a mass range between 0.5 and 3 kDa [12]. Several methods for PRPs purification are available essentially based on a first alcoholic extraction followed by tangential filtration allowing PRP separation, characterized by low MW in comparison to the proteic components characterized by a greater MW.

For example, Sokolowska et al. [13] disclosed a method based on 60% methanol extraction followed by diafiltration using Hollow Fiber with a cut-off of 10 kDa. The obtained filtrate was concentrated on Hollow fiber with a cut off of 1 kDa.

Through LC-ESI-MS analysis and Edman's degradation assay, a set of peptides belonging to PRP's family was identified [12]. The following Table 9 depicts the list of the identified polypeptides and their respective MWs.

TABLE 9: *PRPs nowadays reported in literature*

| PRP | Molecular weight |
|---|---|
| DKE | 390 |
| LNF | 392 |
| SEQP (SEQ ID NO:1) | 459 |
| VVMEV (SEQ ID NO:2) | 575 |
| SEEMP (SEQ ID NO:3) | 591 |
| DSQPPV (SEQ ID NO:4) | 641 |
| CPQSVH (SEQ ID NO:5) | 669 |

(continued)

| PRP | Molecular weight |
|---|---|
| VLPPNVG (SEQ ID NO:6) | 694 |
| DPPPPQS (SEQ ID NO:7) | 736 |
| MQPPPLP (SEQ ID NO:8) | 778 |
| KYKLQE (SEQ ID NO:9) | 807 |
| AFLLYQE (SEQ ID NO:10) | 882 |
| RGPFPILV (SEQ ID NO:11) | 898 |
| SLPQNILPL (SEQ ID NO:12) | 994 |
| VYPFTGPIN (SEQ ID NO:13) | 1006 |
| VESYVPLFP (SEQ ID NO:14) | 1050 |
| TQTPVVVPPF (SEQ ID NO:15) | 1084 |
| QPLPPTVMFP (SEQ ID NO:16) | 1126 |
| SWMHQPPQLP (SEQ ID NO:17) | 1220 |
| MPQNFYKLPQM (SEQ ID NO:18) | 1396 |
| LFFFLPVVNVLP(SEQ ID NO:19) | 1403 |
| AFLLYQEPVLGPV (SEQ ID NO:20) | 1445 |
| MHQPPQPLPPTVMF (SEQ ID NO:21) | 1619 |
| VLEMKFPPPPQETVT (SEQ ID NO:22) | 1712 |
| DLEMPVLPVEPFPFV (SEQ ID NO:23) | 1728 |
| LKPFPKLKVEVFPFP (SEQ ID NO:24) | 1785 |
| LQTPQPLLQVMMEPQGD (SEQ ID NO:25) | 1924 |
| SLTLTDVEKLHLPLPLVQ (SEQ ID NO:26) | 2015 |
| LQPEIMGVPKVKETMVPK (SEQ ID NO:27) | 2023 |
| DQPPDVEKPDLQPFQVQS (SEQ ID NO:28) | 2066 |
| ATFNRYQDDHGEEILKSL (SEQ ID NO:29) | 2135 |
| HKEMPFPKYPVEPFTESQ (SEQ ID NO:30) | 2190 |
| LSQPKVLPVPQKAVPQPDMPIQ (SEQ ID NO:31) | 2409 |

From the above table it may be inferred that less than half of the reported PRPs (42%) have a MW < 1000 Da and more than 82% are characterized by a MW < 2000 Da.

The process for the production of the lactose-free preparate containing mixtures of proteic factors from bovine colostrum of the invention, foresees the diafiltration of the obtained centrifugate, using ultrafiltration membranes with a cut off of 4000 Da. Such operation, as well as to concentrate the centrifugate, allows lactose depletion and more generally the depletion of LMW components, such as sialic acid (MW= 309).

On the basis of the MW distribution of the above reported PRPs it is clear that the above diafiltration step is able to separate PRPs together with LMW components, such as sialic acid. To confirm the above prediction, an LC-ESI-MS/MS analysis of the lactose-free preparation containing the proteic factors from bovine colostrum of the invention (herein indicated as Retentate) and of the permeate obtained from diafiltration and further concentrated by osmosis (herein indicated as Permeate).

LC-ESI-MS analysis has been carried out in MS/MS mode (data dependent scan mode) using a Thermo Finningam Surveyor™ system (ThermoFinningan Italia, Rodano, Milan, Italy) equipped with quaternary pump, thermostated autosampler and under vacuum degasification system connected with a triple quadrupole mass spectrometer Finningan TSQ Quantum Ultra™ (ThermoFinningan Italia, Rodano, Milan, Italy). Data were elaborated using the Xcalibur™ software (ThermoFinningan).

Chromatographic separation was obtained through reverse elution using a Phenomenex Sinergy polar-RP column (150 X 2 mm; particles diameter 4 μm; Chemtek Analitica, Anzola Emilia, Bologna, Italy) protected by a polar-RP precolumn (4 X 2 mm; particles diameter 4 μm; Chemtek Analitica, Anzola Emilia, Bologna, Italy), maintained at a temperature of 25°C by thermostatation. The flow rate of the mobile phase has been maintained at 0.2 ml $min^{-1}$ with linear gradient from 100% of phase A (0.1% formic acid in $H_2O$) to 80% of phase B ($CH_3CN$) in 24 minutes, followed by a period of 6 minutes of isocratic elution. The eluent composition was reported to the initial conditions in 2 minutes with a further equilibrium phase of 6 minutes.

The autosampler was maintained at 4°C by thermostatation, in order to guarantee the optimal conditions of stability during injections (injecting volume 15 μl).

The ESI/MS analysis were carried out in the following experimental conditions: capillary temperature 270°C; spray voltage 4.0 kV; capillary voltage 21 kV. Nebulization gas (nitrogen) was maintained at a flow rate 0.5 L $min^{-1}$.

As reported in Figure 9, the LC-ESI-MS analysis of the permeate shows the elution of several peaks relative to peptides in a nominal mass range comprised between m/z 620 and 2089 in a retention time comprised between 5 to 10 minutes. The MS/MS analysis and the search on database allowed to identify 10 peptides reported in literature as PRPs whose sequences are depicted in Table 10.

TABLE 10. Peptides identified in the permeate

| Peptide | Nominal mass |
|---|---|
| CPQSVH (SEQ ID NO:5) | 669 |
| VLPPNVG (SEQ ID NO:6) | 694 |
| DPPPPQS (SEQ ID NO:7) | 736 |
| MQPPPLP (SEQ ID NO:8) | 778 |
| LFFFLPVVNVLP (SEQ ID NO:19) | 1403 |
| MHQPPQPLPPTVMF (SEQ ID NO:21) | 1619 |
| LKPFPKLKVEVFPFP (SEQ ID NO:24) | 1785 |
| LQTPQPLLQVMMEPQGD (SEQ ID NO:25) | 1924 |
| LQPEIMGVPKVKETMVPK (SEQ ID NO:27) | 2023 |
| DQPPDVEKPDLQPFQVQS (SEQ ID NO:28) | 2066 |

The analysis confirms 10 out 33 of the PRPs reported in literature and the presence of other peptides (at least 12) not yet identified. The partial PRPs overlapping is due to the high variability of the PRPs composition depending on several factors, such as the time of colostrum collection.

This peptide profile was not depicted at all in the retentate, also enhancing the concentration towards the column capacity limit, while elution was observed between 25 and 35 minutes of a protein fraction with MW higher than 12,000 Da, among which lactalbumine and the two isoforms of lactoglobuline, that are absent in the permeate.

The above LC-ESI-MS analysis was carried out in order to exclude the presence of PRPs and sialic acid in the final bovine colostrum extract obtainable according to the process of the invention. The data confirm that the permeate profile contains a peptide pool comprising among the other 10 out 33 of the PRPs disclosed in literature (see Tables 9 and 10). Such peptides are not detectable, as well as sialic acid (PRP detection limit determined with a peptide of synthesis = 1%) in the mixtures of proteic factors from bovine colostrum obtained according to the process of the invention. On the contrary, the mixtures of proteic factors from bovine colostrum obtained according to the process of the invention contain several proteins (absent in the permeate) such as lactoalbumin and the two isoforms of lactoglobulins.

BIBLIOGRAPHY

[1] Alternative Medicine Review, 8(4), 2003, 378.

[2] Int. J. Clin. Pharmacol. and Therapy, 46(5), 2008, 211-225.

[3] Vistoli G., De Maddis D., Cipak A., Zarkovic N., Carini M., Aldini G., "Advanced glycoxidation and lipoxidation end products (AGEs and ALEs): an overview of their mechanisms of formation." Free Radic. Res. August 2013; 47 Suppl 1:3-27.

[4] Aldini G., Dalle-Donne I., Facino R.M., Milzani A., Carini M., "Intervention strategies to inhibit protein carbonylation by lipoxidation-derived reactive carbonyls". Med. Res. Rev. November 2007; 27(6):817-68.
[5] Poulsen M.W., Hedegaard R.V., Andersen J.M., de Courten B., Bügel S., Nielsen J., Skibsted L.H., Dragsted L.O., "Advanced glycation endproducts in food and their effects on health." Food Chem. Toxicol. October 2013; 60:10-37.
[6] Ott C., Jacobs K., Haucke E., Navarrete Santos A., Grune T., Simm A., "Role of advanced glycation end products in cellular signaling". Redox Biol. January 2014; 2:411-29.
[7] Meltretter J., Pischetsrieder M., "Application of mass spectrometry for the detection of glycation and oxidation products in milk proteins." Ann. N.Y. Acad. Sci. April 2008; 1126:134-40.
[8] Fenaille F., Parisod V., Tabet J.C., Guy P.A., "Carbonylation of milk powder proteins as a consequence of processing conditions. Proteomics." August 2005; 5(12):3097-104.
[9] WO2013/098331.
[10] WO2013/098333.
[11] WO2011/064114.
[12] Kruzel ML, Janusz M, Lisowski J, Fischleigh RV, Georgiades JA. J. Mol. Neurosci. 2001 Dec; 17(3):379-89.
[13] Agata Sokoowskaa, Renata Bednarza, Magdalena Pacewicza, Jerzy A. Georgiadesb, Tadeusz Wilusza, Antoni Polanowskia. International Dairy Journal 18 (2008) 204-209.

## Claims

1. Process for the preparation of a mixture of protein factors from bovine colostrum comprising the following steps:

   a) dilution of bovine colostrum, skimming and precipitation of casein at pH between 4 and 6;
   b) centrifugation to remove the lipid and casein components;
   c) concentration and diafiltration on polymer membrane with a molecular cut between 1000 to 20000 Da to reduce the lactose content in the retentate;
   d) adjusting the pH around neutrality and centrifugation;
   e) filtration;
   f) drum filling and freezing;

   wherein the step c) is repeated to obtain a retentate having a lactose content ≤1% calculated with respect to dry weight.

2. Process according to claim 1, wherein the centrifugation of step a) preferably takes place at 4000 rpm.

3. Process according to any one of claims 1-2, wherein the pH of step a) is 4.6, the isoelectric pH of casein.

4. Process according to any one of claims 1-3, wherein in step c) the polymer membrane molecular cut off is 4000 Da.

5. Process according to any one of claims 1-4, wherein the centrifuging of step d) is carried out at 8500 rpm.

6. Process according to any of claims 1-5, wherein the filtration of step e) is carried out on filters of porosity between 0.5 μm and 2 mM to facilitate the subsequent sterilizing filtration carried out with filters of porosity between 0.45 μm and 1 μm.

7. Process according to any preceding claim, further comprising a step g) of freeze-drying the frozen product obtained in step f).

8. A mixture of protein factors from bovine colostrum having a lactose content ≤ 1% calculated with respect to dry weight, obtainable by the process according to any one of claims 1-7.

9. The mixture of protein factors from bovine colostrum according to claim 8, for use as a medicament.

10. The mixture of protein factors from bovine colostrum for use according to claim 9, which is administered topically or orally.

11. The mixture of protein factors from bovine colostrum for use according to claim 10, for use in the treatment of pathological conditions that require the tissue healing or regeneration, said pathological condition being preferably selected from the group comprising skin lesions or ulcers, burns, oral lesions, corneal lesions, nasal mucosa lesions, lesions caused by Herpes virus and for the healing of wounds.

12. Use of the mixture of protein factors from bovine colostrum according to claim 8 as food supplement.

## Patentansprüche

1. Verfahren zur Herstellung einer Mischung von Proteinfaktoren aus Rinderkolostrum, umfassend die folgenden Schritte:

   a) Verdünnen von Rinderkolostrum, Abschöpfen und Ausfällen von Kasein bei einem pH-Wert zwischen 4 und 6;
   b) Zentrifugieren zum Entfernen der Lipid- und Kaseinbestandteile;
   c) Konzentration und Diafiltration auf einer Polymermembran mit einem molekularen Schnitt zwischen 1000 und 20000 Da zur Verminderung des Laktosegehalts im Retentat;
   d) Einstellen auf einen etwa neutralen pH-Wert und Zentrifugieren;
   e) Filtrieren;
   f) Trommelbefüllung und Einfrieren;

wobei der Schritt c) wiederholt wird, um ein Retentat mit einem auf das Trockengewicht berechneten Laktosegehalt ≤1% zu erhalten.

2. Verfahren nach Anspruch 1, wobei das Zentrifugieren in Schritt a) vorzugsweise bei 4000 U/min ausgeführt wird.

3. Verfahren nach einem der Ansprüche 1-2, wobei der pH-Wert in Schritt a) bei 4,6, dem isoelektrischen pH-Wert von Kasein liegt.

4. Verfahren nach einem der Ansprüche 1-3, wobei der molekulare Schnitt der Polymermembran in Schritt c) bei 4000 Da liegt.

5. Verfahren nach einem der Ansprüche 1-4, wobei das Zentrifugieren in Schritt d) bei 8500 U/min ausgeführt wird.

6. Verfahren nach einem der Ansprüche 1-5, wobei das Filtrieren in Schritt e) auf Filtern mit einer Porosität zwischen 0,5 μm und 2 mM ausgeführt wird, um die anschließende Sterilfiltration zu erleichtern, die mit Filtern mit einer Porosität zwischen 0,45 μm und 1 μm ausgeführt wird.

7. Verfahren nach einem der vorstehenden Ansprüche, ferner umfassend einen Schritt g) des Geriertrocknens des in Schritt f) erhaltenen, gefrorenen Produkts.

8. Mischung von Proteinfaktoren aus Rinderkolostrum mit einem auf das Trockengewicht berechneten Laktosegehalt von ≤ 1%, die durch das Verfahren nach einem der Ansprüche 1-7 erhältlich ist.

9. Mischung von Proteinfaktoren aus Rinderkolostrum nach Anspruch 8 zur Verwendung als ein Medikament.

10. Mischung von Proteinfaktoren aus Rinderkolostrum zur Verwendung nach Anspruch 9, die topisch oder oral verabreicht wird.

11. Mischung von Proteinfaktoren aus Rinderkolostrum zur Verwendung nach Anspruch 10, zur Verwendung bei der Behandlung pathologischer Zustände, die die Heilung oder Regenerierung von Gewebe erfordern, wobei die pathologischen Zustände vorzugsweise ausgewählt sind aus der Gruppe umfassend Hautläsionen oder Geschwüre, orale Läsionen, Hornhautläsionen, Läsionen der Nasenschleimhaut, durch Herpesviren verursachte Läsionen und für die Wundheilung.

12. Mischung von Proteinfaktoren aus Rinderkolostrum nach Anspruch 8 als Nahrungsergänzungsmittel.

**Revendications**

1. Procédé de préparation d'un mélange de facteurs protéiques à partir de colostrum bovin comprenant les étapes suivantes :

   a) dilution de colostrum bovin, écrémage et précipitation de la caséine à un pH entre 4 et 6 ;
   b) centrifugation pour retirer les constituants lipidiques et de caséine ;
   c) concentration et diafiltration sur membrane polymère ayant une coupure moléculaire entre 1 000 et 20 000 Da pour réduire la teneur en lactose dans le rétentat ;
   d) ajustement du pH autour de la neutralité et centrifugation ;
   e) filtration ;
   f) remplissage en cuves et congélation ;

   dans lequel l'étape c) est répétée pour obtenir un rétentat ayant une teneur en lactose ≤ 1 % calculée par rapport au poids sec.

2. Procédé selon la revendication 1, dans lequel la centrifugation de l'étape a) se produit de préférence à 4 000 tr/min.

3. Procédé selon l'une quelconque des revendications 1 à 2, dans lequel le pH de l'étape a) est de 4,6, le pH isoélectrique de la caséine.

4. Procédé selon l'une quelconque des revendications 1 à 3, dans lequel à l'étape c) la coupure moléculaire de membrane polymère est de 4 000 Da.

5. Procédé selon l'une quelconque des revendications 1 à 4, dans lequel la centrifugation de l'étape d) est réalisée à 8 500 tr/min.

6. Procédé selon l'une quelconque des revendications 1 à 5, dans lequel la filtration de l'étape e) est réalisée sur des filtres de porosité entre 0,5 μm et 2 mM pour faciliter la filtration stérilisante ultérieure réalisée avec des filtres de porosité entre 0,45 μm et 1 μm.

7. Procédé selon une quelconque revendication précédente, comprenant en outre une étape g) de lyophilisation du produit congelé obtenu à l'étape f).

8. Mélange de facteurs protéiques à partir de colostrum bovin ayant une teneur en lactose ≤ 1 % calculée par rapport au poids sec, apte à être obtenu par le procédé selon l'une quelconque des revendications 1 à 7.

9. Mélange de facteurs protéiques à partir de colostrum bovin selon la revendication 8, pour une utilisation comme médicament.

10. Mélange de facteurs protéiques à partir de colostrum bovin pour une utilisation selon la revendication 9, qui est administré par voie topique ou orale.

11. Mélange de facteurs protéiques à partir de colostrum bovin pour une utilisation selon la revendication 10, pour une utilisation dans le traitement d'états pathologiques qui nécessitent la guérison ou la régénération de tissus, ledit état pathologique étant de préférence sélectionné parmi le groupe comprenant les lésions ou les ulcères de la peau, les brûlures, les lésions buccales, les lésions cornéennes, les lésions de la muqueuse nasale, les lésions provoquées par le virus de l'herpès et pour la cicatrisation des plaies.

12. Utilisation du mélange de facteurs protéiques à partir de colostrum bovin selon la revendication 8 comme complément alimentaire.

General scheme of the process:

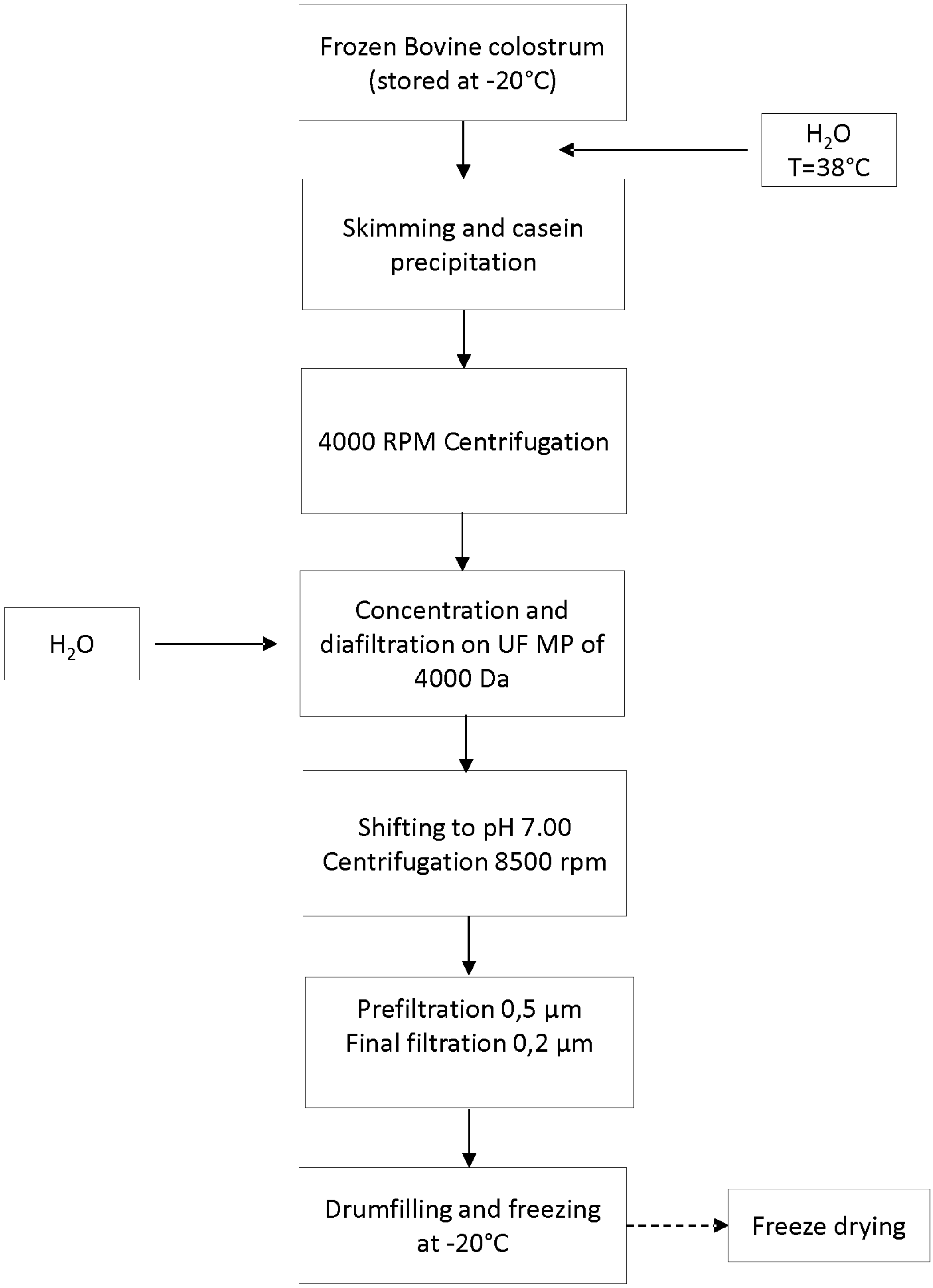


Fig. 1

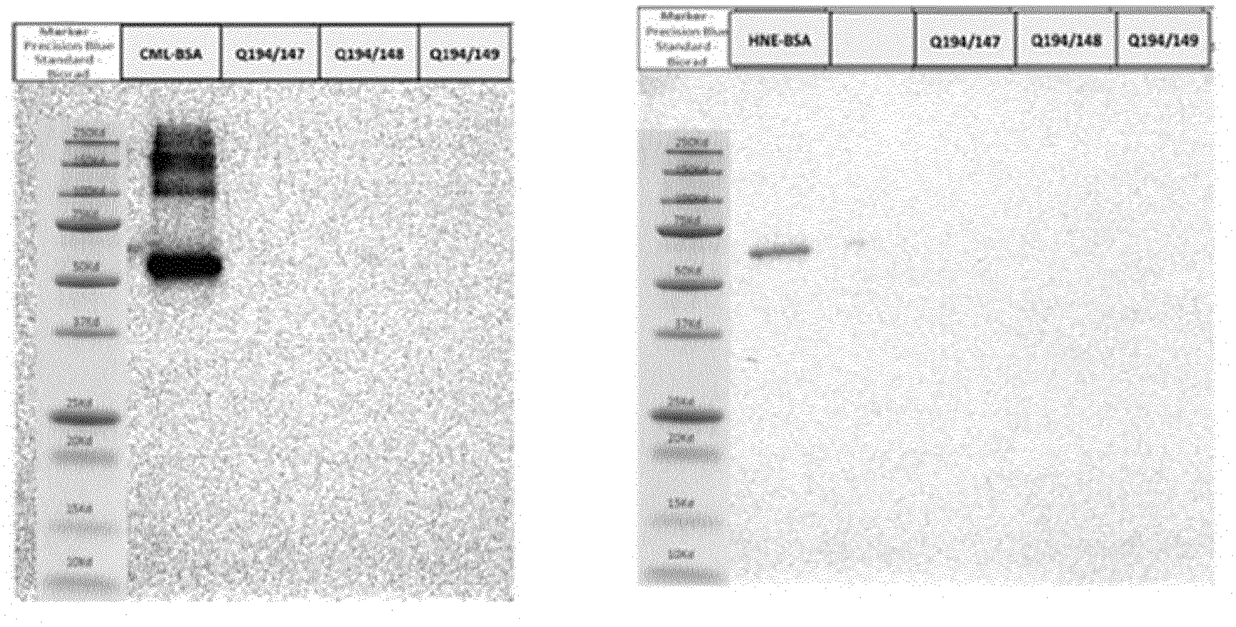
CML-BSA
Q194/147
Q194/148
Q194/149
HNE-BSA
Q194/147
Q194/148
Q194/149

Fig. 2

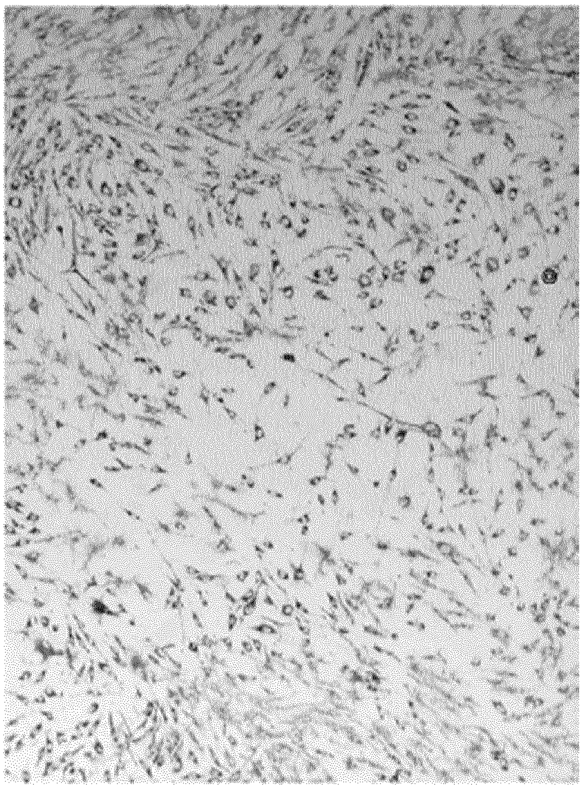

Fig. 3b

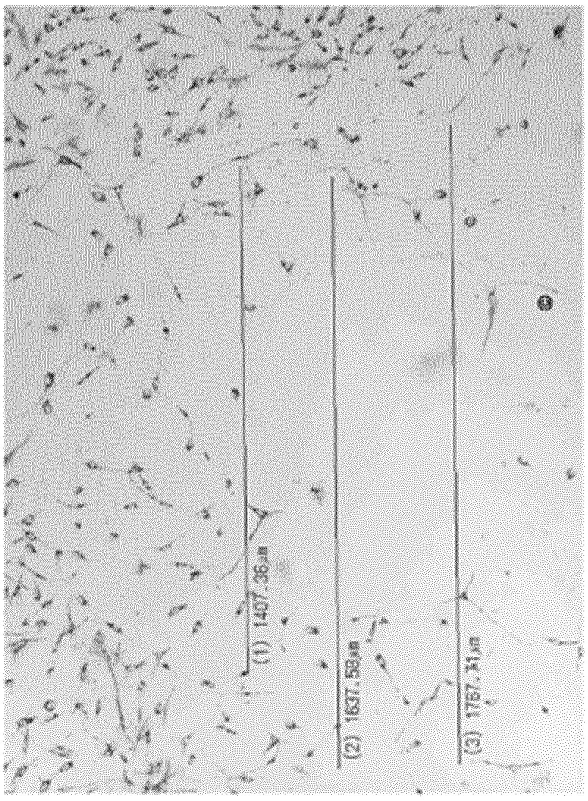


Fig. 3a

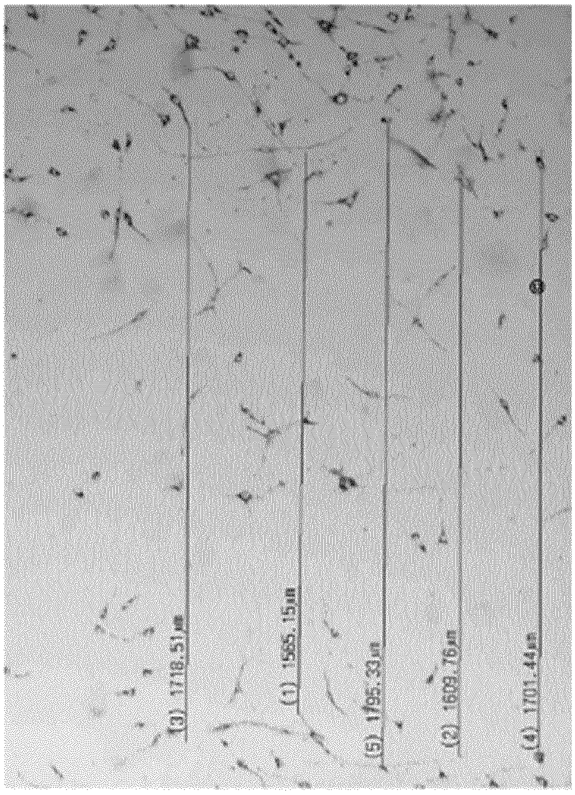


Fig. 3c

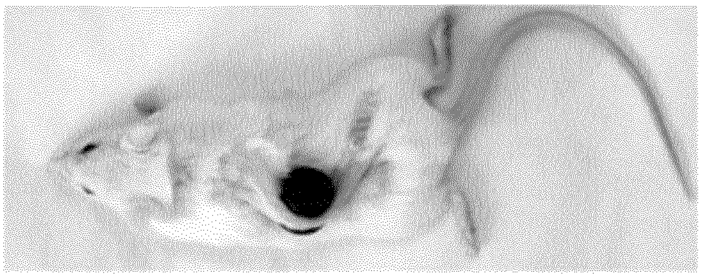

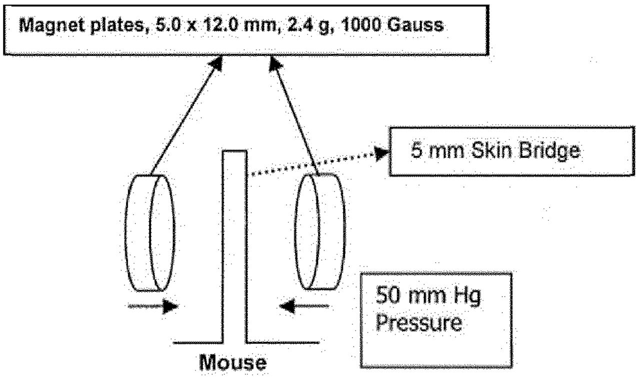
Magnet plates, 5.0 x 12.0 mm, 2.4 g, 1000 Gauss
5 mm Skin Bridge
50 mm Hg
Pressure
Mouse

Fig. 4

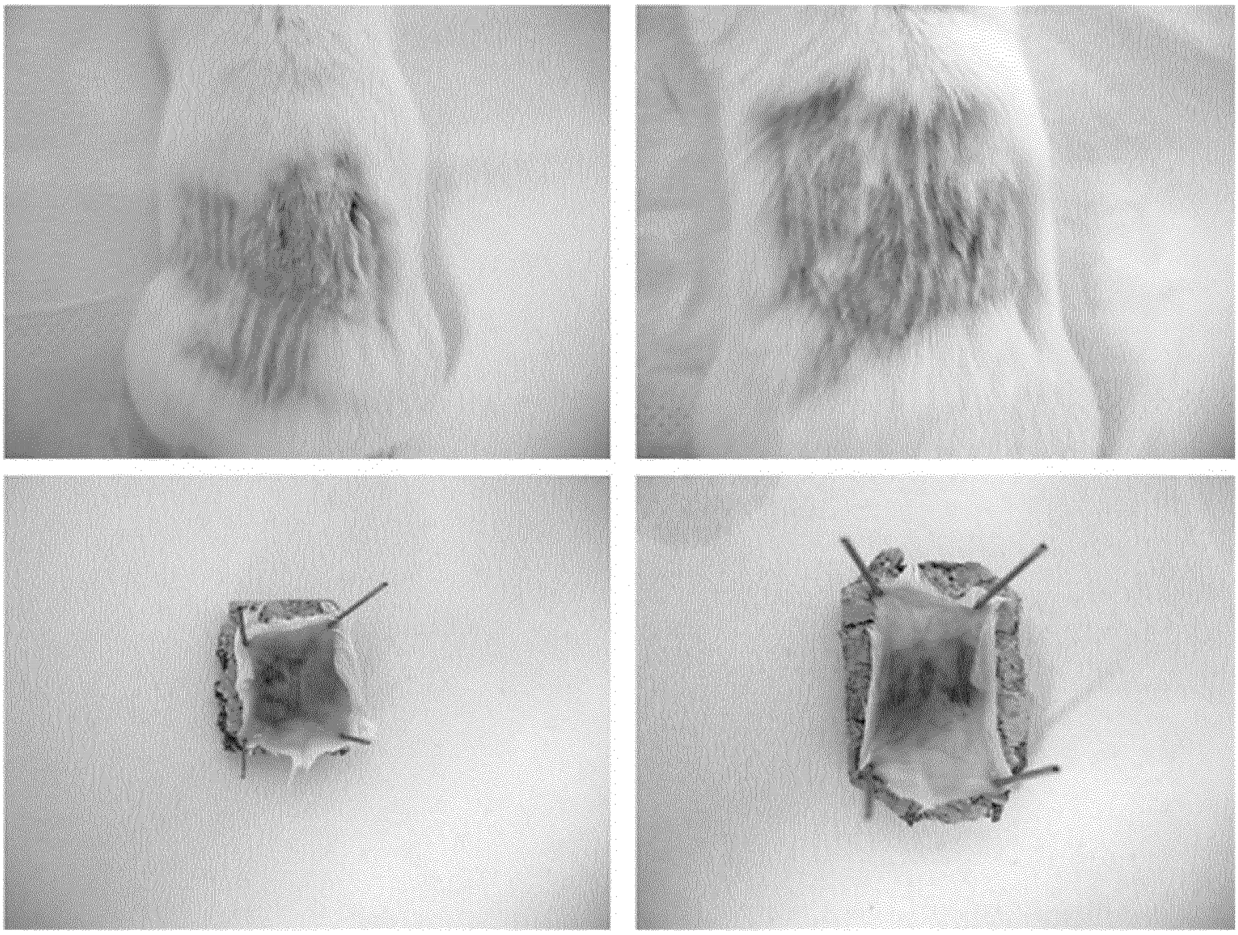

Fig. 5

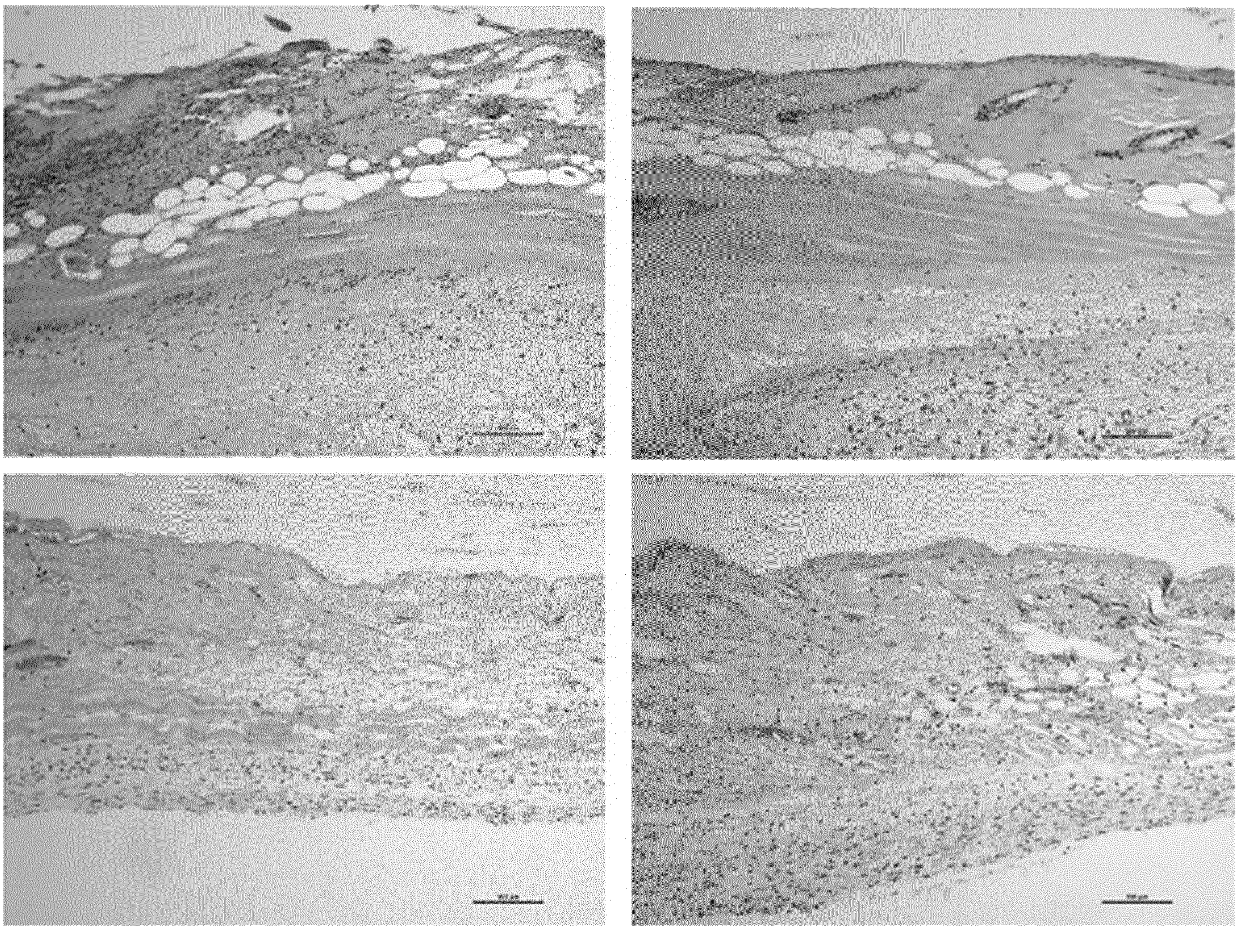

Fig. 6

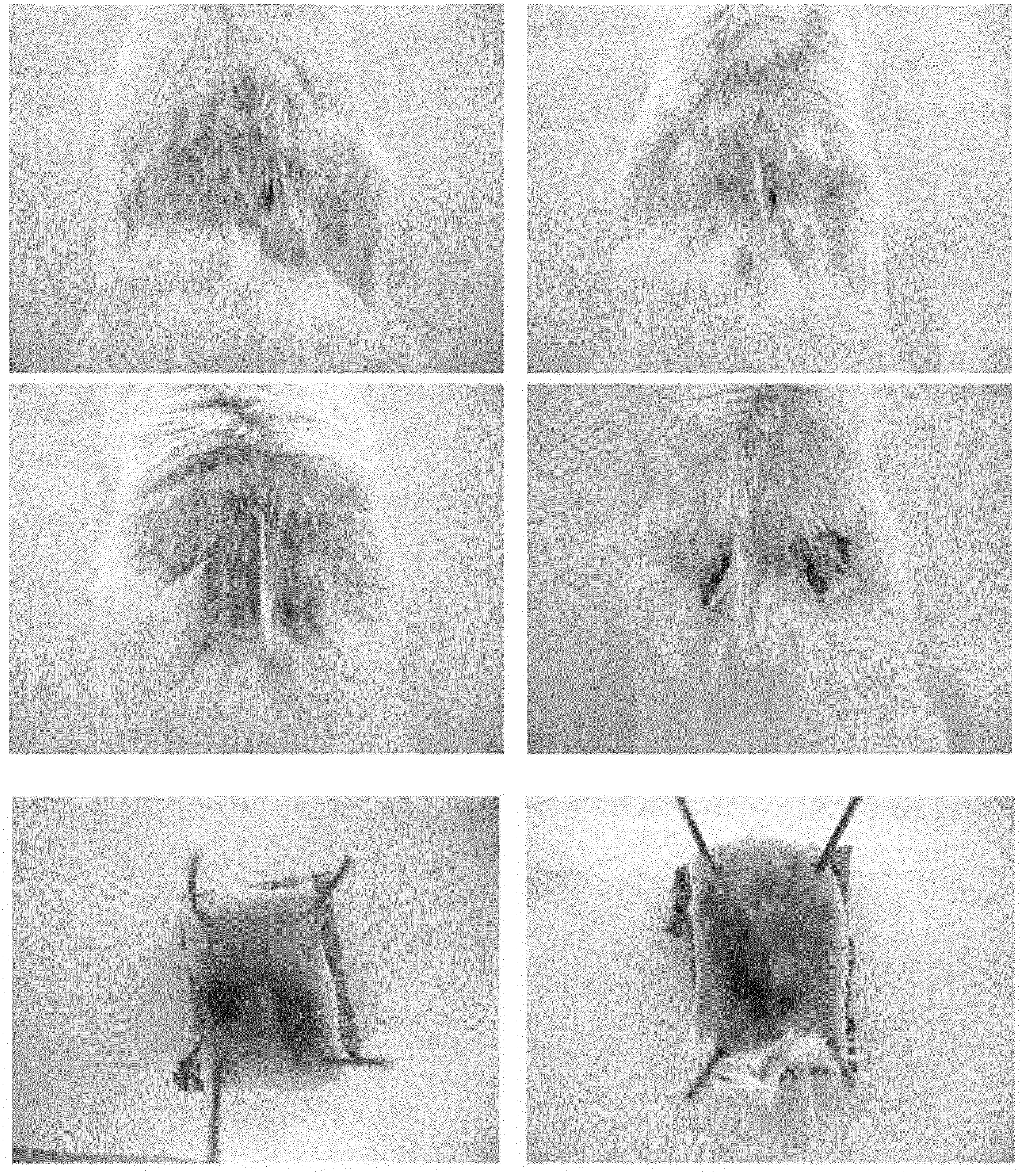

Fig. 7

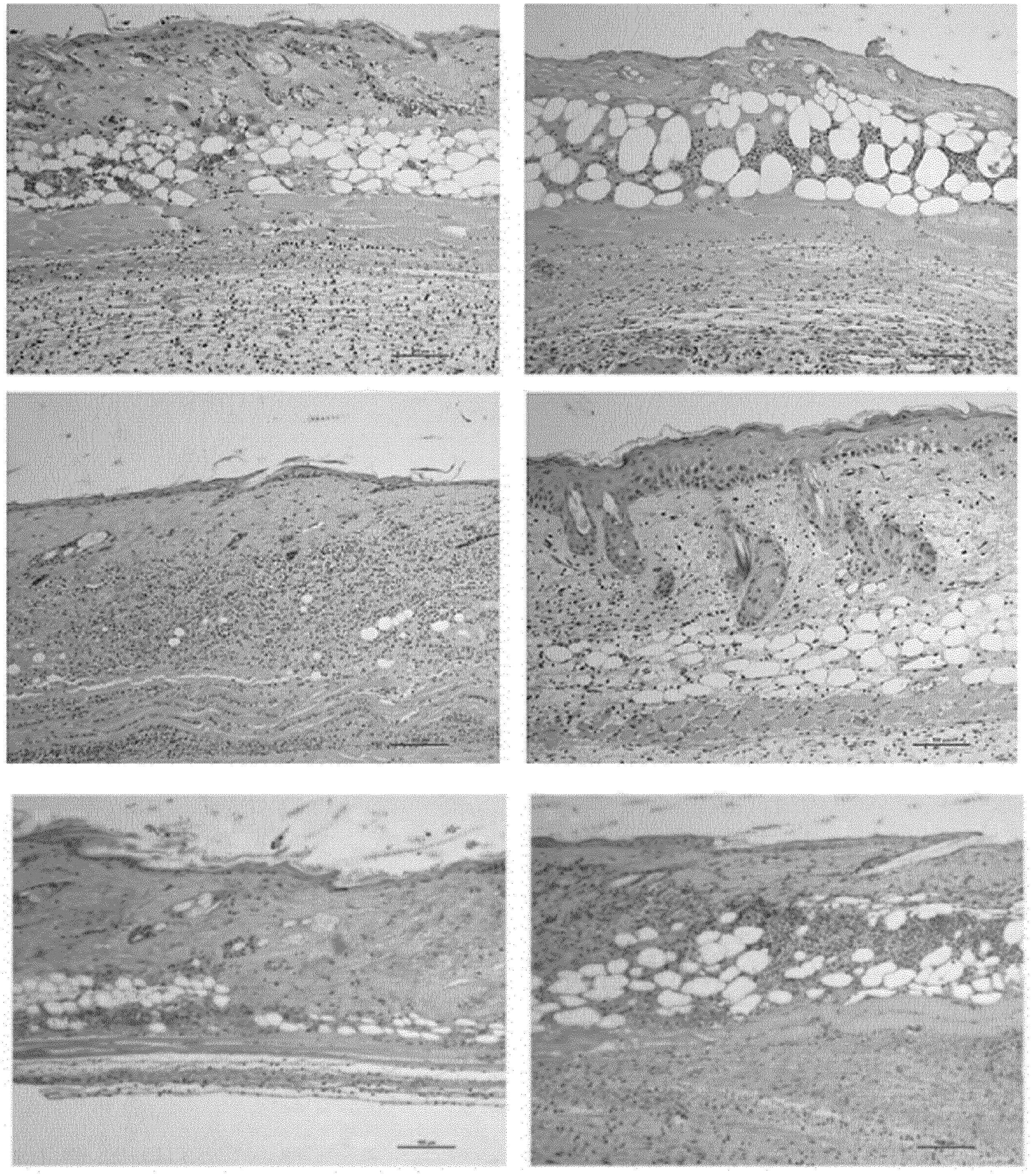

Fig. 8

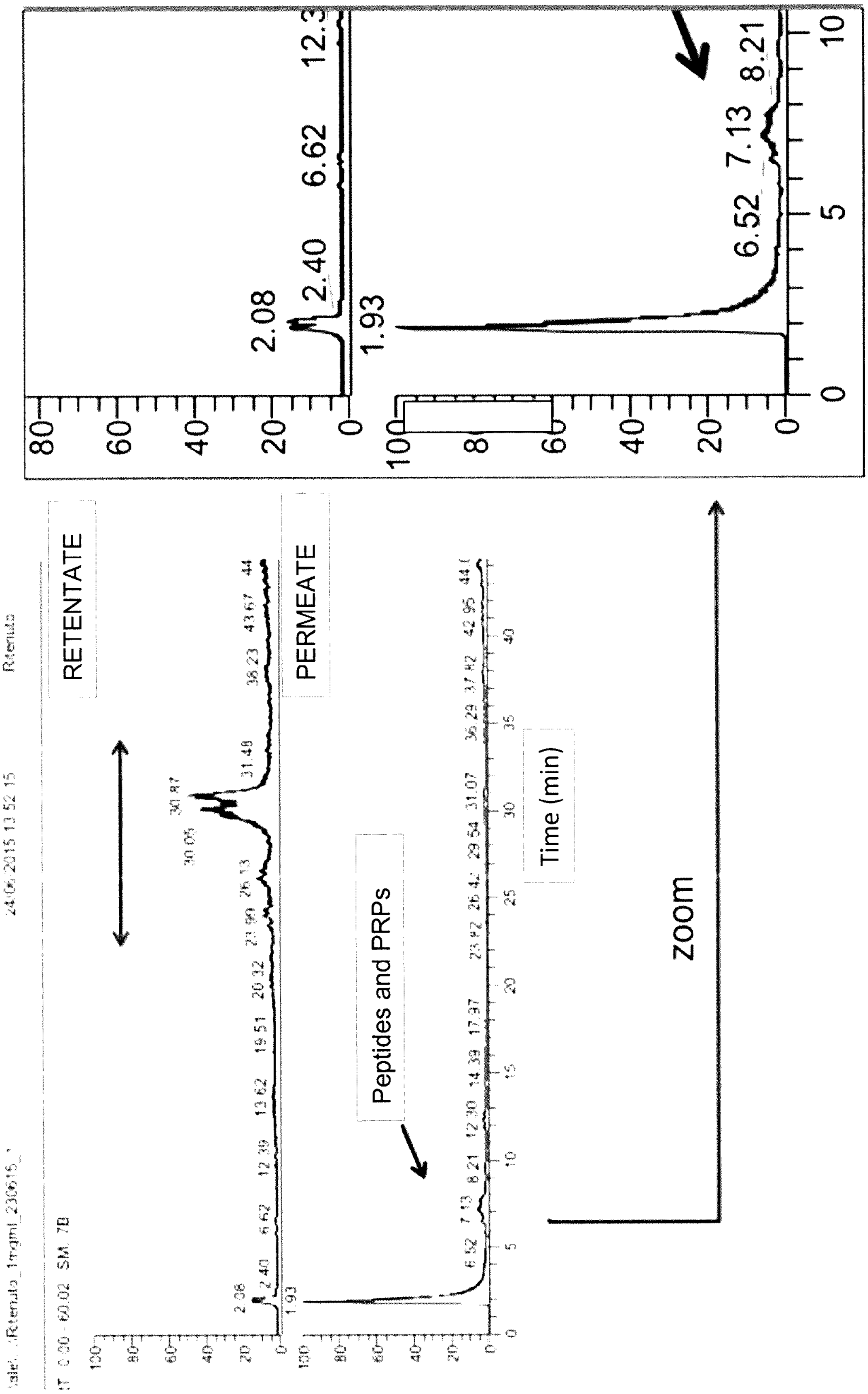


Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013098331 A
- WO 2013098333 A
- WO 2011064114 A
- WO 2012087165 A
- WO 2009135306 A

### Non-patent literature cited in the description


- *Alternative Medicine Review,* 2003, vol. 8 (4), 378
- *Int. J. Clin. Pharmacol. and Therapy,* 2008, vol. 46 (5), 211-225
- **VISTOLI G. ; DE MADDIS D. ; CIPAK A. ; ZARKOVIC N. ; CARINI M. ; ALDINI G.** Advanced glycoxidation and lipoxidation end products (AGEs and ALEs): an overview of their mechanisms of formation. *Free Radic. Res.,* August 2013, vol. 47 (1), 3-27
- **ALDINI G. ; DALLE-DONNE I. ; FACINO R.M. ; MILZANI A. ; CARINI M.** Intervention strategies to inhibit protein carbonylation by lipoxidation-derived reactive carbonyls. *Med. Res. Rev.,* November 2007, vol. 27 (6), 817-68
- **POULSEN M.W. ; HEDEGAARD R.V. ; ANDERSEN J.M. ; DE COURTEN B. ; BÜGEL S. ; NIELSEN J. ; SKIBSTED L.H. ; DRAGSTED L.O.** Advanced glycation endproducts in food and their effects on health. *Food Chem. Toxicol.,* October 2013, vol. 60, 10-37
- **OTT C. ; JACOBS K. ; HAUCKE E. ; NAVARRETE SANTOS A. ; GRUNE T. ; SIMM A.** Role of advanced glycation end products in cellular signaling. *Redox Biol.,* January 2014, vol. 2, 411-29
- **MELTRETTER J. ; PISCHETSRIEDER M.** Application of mass spectrometry for the detection of glycation and oxidation products in milk proteins. *Ann. N.Y. Acad. Sci.,* April 2008, vol. 1126, 134-40
- **FENAILLE F. ; PARISOD V. ; TABET J.C. ; GUY P.A.** Carbonylation of milk powder proteins as a consequence of processing conditions. *Proteomics,* August 2005, vol. 5 (12), 3097-104
- **KRUZEL ML ; JANUSZ M ; LISOWSKI J ; FISCHLEIGH RV ; GEORGIADES JA.** *J. Mol. Neurosci.,* December 2001, vol. 17 (3), 379-89
- **AGATA SOKOOWSKAA ; RENATA BEDNARZA ; MAGDALENA PACEWICZA ; JERZY A. GEORGIADESB ; TADEUSZ WILUSZA ; ANTONI POLANOWSKIA.** *International Dairy Journal,* 2008, vol. 18, 204-209

SEQUENCE LISTING

<110> TENAGRO s.r.l.

<120> PROCESS FOR THE PREPARATION OF HIGH PURITY MIXTURES OF PROTEIN FACTORS FROM BOVINE COLOSTRUM

<130> P1176PC

<150> MI2014A001450
<151> 2014-08-06

<160> 31

<170> PatentIn version 3.5

<210> 1
<211> 4
<212> PRT
<213> Artificial

<220>
<223> Proline rich peptide

<400> 1

```
Ser Glu Gln Pro
1
```

<210> 2
<211> 5
<212> PRT
<213> Artificial

<220>
<223> Proline rich peptide

<400> 2

Val Val Met Glu Val
1 5

<210> 3
<211> 5
<212> PRT
<213> Artificial

<220>
<223> Proline rich peptide

<400> 3

Ser Glu Glu Met Pro
1 5

<210> 4
<211> 6
<212> PRT
<213> Artificial

<220>
<223> Proline rich peptide

<400> 4

Asp Ser Gln Pro Pro Val
1 5

<210> 5
<211> 6
<212> PRT
<213> Artificial

<220>
<223> Proline rich peptide

<400> 5

Cys Pro Gln Ser Val His
1 5

<210> 6
<211> 7
<212> PRT
<213> Artificial

<220>
<223> Proline rich peptide

<400> 6

Val Leu Pro Pro Asn Val Gly
1 5

<210> 7

<211> 7
<212> PRT
<213> Artificial

<220>
<223> Proline rich peptide

<400> 7

```
Asp Pro Pro Pro Pro Gln Ser
1               5
```

<210> 8
<211> 7
<212> PRT
<213> Artificial

<220>
<223> Proline rich peptide

<400> 8

```
Met Gln Pro Pro Pro Leu Pro
1               5
```

<210> 9
<211> 6
<212> PRT
<213> Artificial

<220>
<223> Proline rich peptide

<400> 9

```
Lys Tyr Lys Leu Gln Glu
1               5
```

<210> 10
<211> 7
<212> PRT
<213> Artificial

<220>
<223> Proline rich peptide

<400> 10

```
Ala Phe Leu Leu Tyr Gln Glu
1               5
```

<210> 11
<211> 8
<212> PRT
<213> Artificial

<220>
<223> Proline rich peptide

<400> 11

Arg Gly Pro Phe Pro Ile Leu Val
1 5

<210> 12
<211> 9
<212> PRT
<213> Artificial

<220>
<223> Proline rich peptide

<400> 12

Ser Leu Pro Gln Asn Ile Leu Pro Leu
1 5

<210> 13
<211> 9
<212> PRT
<213> Artificial

<220>
<223> Proline rich peptide

<400> 13

Val Tyr Pro Phe Thr Gly Pro Ile Asn
1 5

<210> 14
<211> 9
<212> PRT
<213> Artificial

<220>
<223> Proline rich peptide

<400> 14

Val Glu Ser Tyr Val Pro Leu Phe Pro
1 5

<210> 15
<211> 10
<212> PRT
<213> Artificial

<220>
<223> Proline rich peptide

<400> 15

Thr Gln Thr Pro Val Val Val Pro Pro Phe
1 5 10

<210> 16

<211> 10
<212> PRT
<213> Artificial

<220>
<223> Proline rich peptide

<400> 16

```
Gln Pro Leu Pro Pro Thr Val Met Phe Pro
1               5                   10
```

<210> 17
<211> 10
<212> PRT
<213> Artificial

<220>
<223> Proline rich peptide

<400> 17

```
Ser Trp Met His Gln Pro Pro Gln Leu Pro
1               5                   10
```

<210> 18
<211> 11
<212> PRT
<213> Artificial

<220>
<223> Proline rich peptide

<400> 18

```
Met Pro Gln Asn Phe Tyr Lys Leu Pro Gln Met
1               5                   10
```

<210> 19
<211> 12
<212> PRT
<213> Artificial

<220>
<223> Proline rich peptide

<400> 19

```
Leu Phe Phe Phe Leu Pro Val Val Asn Val Leu Pro
1               5                   10
```

<210> 20
<211> 13
<212> PRT
<213> Artificial

<220>
<223> Proline rich peptide

<400> 20

Ala Phe Leu Leu Tyr Gln Glu Pro Val Leu Gly Pro Val
1               5                   10

<210> 21
<211> 14
<212> PRT
<213> Artificial

<220>
<223> Proline rich peptide

<400> 21

Met His Gln Pro Pro Gln Pro Leu Pro Pro Thr Val Met Phe
1               5                   10

<210> 22
<211> 15
<212> PRT
<213> Artificial

<220>
<223> Proline rich peptide

<400> 22

Val Leu Glu Met Lys Phe Pro Pro Pro Pro Gln Glu Thr Val Thr
1               5                   10                  15

<210> 23
<211> 15
<212> PRT
<213> Artificial

<220>
<223> Proline rich peptide

<400> 23

Asp Leu Glu Met Pro Val Leu Pro Val Glu Pro Phe Pro Phe Val
1               5                   10                  15

<210> 24
<211> 15
<212> PRT
<213> Artificial

<220>
<223> Proline rich peptide

<400> 24

Leu Lys Pro Phe Pro Lys Leu Lys Val Glu Val Phe Pro Phe Pro
1               5                   10                  15

<210> 25

<211> 17
<212> PRT
<213> Artificial

<220>
<223> Proline rich peptide

<400> 25

```
Leu Gln Thr Pro Gln Pro Leu Leu Gln Val Met Met Glu Pro Gln Gly
1               5                   10                  15

Asp
```

<210> 26
<211> 18
<212> PRT
<213> Artificial

<220>
<223> Proline rich peptide

<400> 26

```
Ser Leu Thr Leu Thr Asp Val Glu Lys Leu His Leu Pro Leu Pro Leu
1               5                   10                  15

Val Gln
```

<210> 27
<211> 18
<212> PRT
<213> Artificial

<220>
<223> Proline rich peptide

<400> 27

```
Leu Gln Pro Glu Ile Met Gly Val Pro Lys Val Lys Glu Thr Met Val
1               5                   10                  15

Pro Lys
```

<210> 28
<211> 18
<212> PRT
<213> Artificial

<220>
<223> Proline rich peptide

<400> 28

Asp Gln Pro Pro Asp Val Glu Lys Pro Asp Leu Gln Pro Phe Gln Val
1               5                   10                  15

Gln Ser

<210> 29
<211> 18
<212> PRT
<213> Artificial

<220>
<223> Proline rich peptide

<400> 29

Ala Thr Phe Asn Arg Tyr Gln Asp Asp His Gly Glu Glu Ile Leu Lys
1               5                   10                  15

Ser Leu

<210> 30
<211> 18
<212> PRT
<213> Artificial

<220>
<223> Proline rich peptide

<400> 30

His Lys Glu Met Pro Phe Pro Lys Tyr Pro Val Glu Pro Phe Thr Glu
1               5                   10                  15

Ser Gln

<210> 31
<211> 22
<212> PRT
<213> Artificial

<220>
<223> Proline rich peptide

<400> 31

Leu Ser Gln Pro Lys Val Leu Pro Val Pro Gln Lys Ala Val Pro Gln
1               5                   10                  15

Pro Asp Met Pro Ile Gln
            20